# EUROPEAN PATENT APPLICATION

(11) **EP 2 735 275 A1**
(43) Date of publication of application: **28.05.2014**
(21) Application number: 12818208.6
(22) Date of filing: 20.07.2012
(51) Int. Cl.: A61B 17/34

(54) **LAPAROSCOPIC PORT**

(30) Priority: 22.07.2011 ES 201130801
(71) Applicant: Servicio Andaluz de Salud, 41071 Sevilla (ES)
(72) Inventor: RUIZ RABELO, Juan Francisco, E-41092 Sevilla (ES); RUFIAN PEÑA, Sebastián, E-41092 Sevilla (ES); SÁNCHEZ HIDALGO, Juan Manuel, E-41092 Sevilla (ES)
(74) Representative: Illescas, Manuel
(86) International application number: PCT/ES2012/070556
(87) International publication number: WO 2013/014313

(57) **Abstract**

The object of the present invention is port for performing laparoscopic surgery operations with a novel arrangement, as compared to currently known ports. Said port is preferably formed by two separate parts which are joined in a seal tight form: a Wall protector and a distensible entry part able to couple with the protector, and which has several openings for the entry of instruments

## Description

### FIELD OF THE INVENTION

The present invention relates to a port for performing laparoscopic surgery operations with a novel and improved arrangement, as compared to currently known ports.

### BACKGROUND OF THE INVENTION

Laparoscopic surgery is a minimally invasive surgical technique in which the practitioner performs the operation through a small incision of a few centimeters. In order to maintain the incision open enough for inserting different instruments into it, several devices or ports are currently known.

One first group of devices is provided with several differentiated work channels for the insertion of instruments. However, these devices suffer from the drawback of limiting the movement degrees of the instruments inside them, increasing the friction and making the material angulation difficult. Moreover, because being made in a single piece, it is necessary to remove the wall device, (part which keeps the incision walls separated) for the removal of specimens and surgical parts from the inside. One example of this type of devices is the so-called "SILS" by the company Covidien.

There is a second type of devices which have a single main work channel and a wall protection part in the form of a double ring. This device has an internal flexible ring and an external one, of non-flexible material, being the work channels directly coupled to the external without leaving enough space for the triangulation, as occurs with Octoport and Triport models. Moreover, this device is made in an internal flexible non slipping material, and which can thus difficult the entry and exit of instrumental. For this reason it is necessary to couple another part, with funnel shape, which protects the internal face of the main channel (Octoport).

None of the devices disclosed to date allows withstanding the intraabdominal pressure created by CO₂ insufflation (necessary for creating the pneumoperitoneum).

### DESCRIPTION OF THE INVENTION

The invention describes a port for single incision laparoscopy formed by two separate parts which are joined in a seal tight form: a wall protector and a distensible entry part able to couple with the protector, and which has several openings for the entry of instruments.

This port solves the abovementioned problems mainly thanks to the fact that the entry part is made of a flexible or distensible material, capable of swelling like a balloon, when it is subject to the intraabdominal pressure applied to obtain the pneumoperitoneum. That is, it is much more flexible than the material of prior art devices, which keeps their form when they are subject to the pneumoperitoneum pressure. The entry piece of the invention, upon swelling, provides a bigger room for maneuver for instruments introduced through the port, which can thus move more easily and with a higher angulation. One further advantage of the port of the invention is that, since it is formed by two separate parts, it allows the removal or extraction of specimens or biological material resulting from the interventions in an easy and convenient way.

The two parts forming the laparoscopy port of the present invention will be described in more detail below.

### Wall protector

The wall protector is formed by a deformable ring and stiff ring joined together by a sleeve of flexible and puncture-proof material. This sleeve is firmly joined to the deformable ring, but in a slidable manner to the stiff ring, with the object of adjusting to the different measures, both in diameter and depth, of the abdominal wall of each patient.

The sleeve's flexible material has to be hypoallergenic and latex-free, since it is going to contact with the patient's skin and tissues. A flexible material based in plastic, polyurethane, polyethylene, nitrile, silicone or others, is preferably used. The expression "puncture-proof" is intended to mean that the material has to be resistant enough as not to puncturing or tearing easily in the event of receiving scratches or punctures during the introduction of the surgical instruments. The thickness of the sleeve will depend on the material chosen in each case, although it will preferably be between 2 mm and 4 mm.

Regarding the size of the ring of this wall protector, they preferably have a diameter of between 6 cm and 10 cm, more preferably 9 cm.

For installing this wall protector, the ring is firstly deformed until it has a size which allows it to be inserted into the abdominal wall opening, typically of between 1,5-3 cm. Once inside, the ring recovers its original shape, the stiff ring remains outside, in such a way that the flexible sleeve exerts traction on the tissues along the radial exterior direction, tending to open them softly for allowing the insertion of surgical material. According to a preferred embodiment of the invention, the stiff ring comprises means for tensioning the sleeve when the protector has been installed in position, thus being able to open the opening for giving way to surgical instruments.

### Entry part

The entry part is made of a flexible material capable of swelling by the injection of a gas at the usual pressures for a performing pneumoperitoneum (usually between 10 mmHg and 20 mmHg), and comprises several valves for the insertion of surgical material. One of the main advantages of the present invention is that the flexible or distensible material used for the entry part is much more flexible than those used in the prior art, with a flexibility similar to that of a balloon or a surgical glove. By using this entry part, the pressure applied to obtain the pneumoperitoneum "swells" the entry part in a way similar to a balloon, giving its final shape during use, and allowing instruments inserted into valves to have a greater freedom of movement, as well as angulation. In principle, the entry part could be made of any material, with the proviso that it meets the described flexibility characteristics, and that it is also puncture-proof during the insertion of surgical instrumental. Preferably, this flexible material is based in plastic, polyurethane, polyethylene, nitrile, silicone or others. Its thickness will depend on the material chosen in each case, although it will preferably be between 1 mm and 3 mm.

This entry part is able to be coupled to the rigid ring of the wall protector in a seal tight form, thus preventing, the exit of gases used for performing the performing pneumoperitoneum. To this end, a threaded or a pressure connection is preferably used. Moreover, in a preferred embodiment of the invention one of the valves comprises a universal female terminal, suitable for the connection of a laparoscopy insufflator.

According to a preferred embodiment, the entry part particularly comprises four valves, more preferably of the following dimensions: three universal valves for material between 5 and 12 mm, and a valve for material between 10 and 15 mm. Universal valves of 5-12 mm. comprise a 12 mm cylinder with a valvular system which prevents the exit of CO₂ and into which instrumental between 5 a 12 mm can be inserted. Correspondingly, universal valves of 10 to 15 mm allow the use of material of between 10 and 15 mm, as optical systems and endostaplers.

### BRIEF DESCRIPTION OF FIGURES

Fig. 1 shows a laparoscopy port according to one example of the present invention.
Fig. 2 shows an example of wall protector according to the invention.
Fig. 3 shows an example of entry part according to the invention.
Figs. 4a-4c show the main steps, necessary for the installation of the laparoscopy port of the invention.

### PREFERRED EMBODIMENT OF THE INVENTION

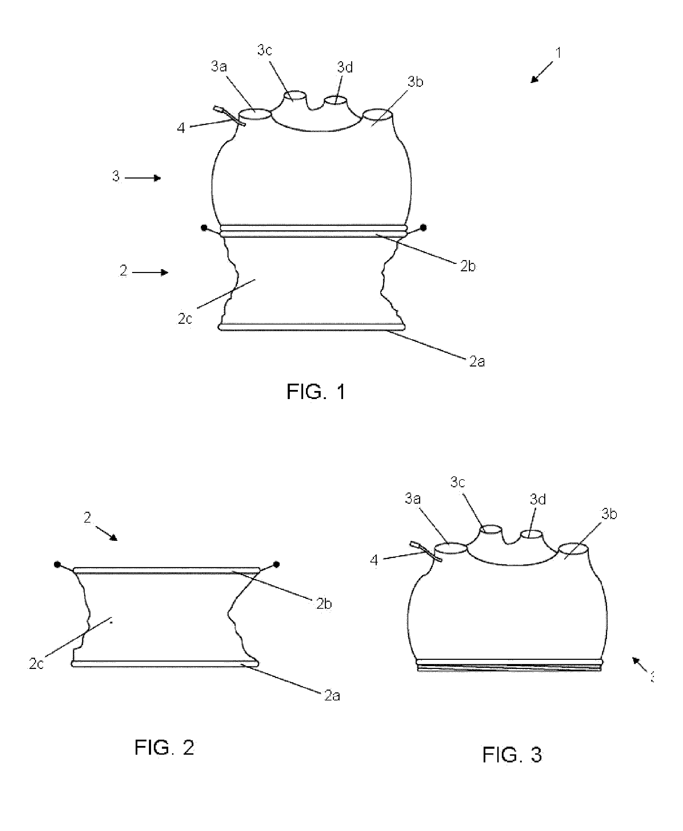

The invention will be described below, with reference to the attached figures. Particularly, Fig. 1 shows an example of a laparoscopy port (1) according to the invention in which its different constituent parts, which are in turn separately represented in Figs. 2 and 3, can be seen.

The wall protector (2) is formed by a flexible or deformable ring (2a), a stiff ring (2b) and a flexible sleeve (2c), firmly joined to the deformable ring (2a) and joined, in a slidable manner, to the stiff ring (2b). Moreover, this example comprises tensioning means, which have been represented as two cords or cables joined to the sleeve (2c) and which pass through the stiff ring (2b) for tensioning the flexible sleeve (2c) once positioned.

The entry part (3) is made, on the other hand, of a flexible material as a balloon, and comprises several valves (3a, 3b, 3c, 3d) for the entry of surgical material. In the example of the figures, four valves have been schematically represented: three valves (3a, 3b, 3c) with a diameter for material of 5 to 12 mm, and a fourth valve (3d) for material of 10 to 15 mm (endostaplers). In this example, the pneumoperitoneum is performed through a female universal terminal (4) for a laparoscopy insufflator which is coupled to the valve (3a).

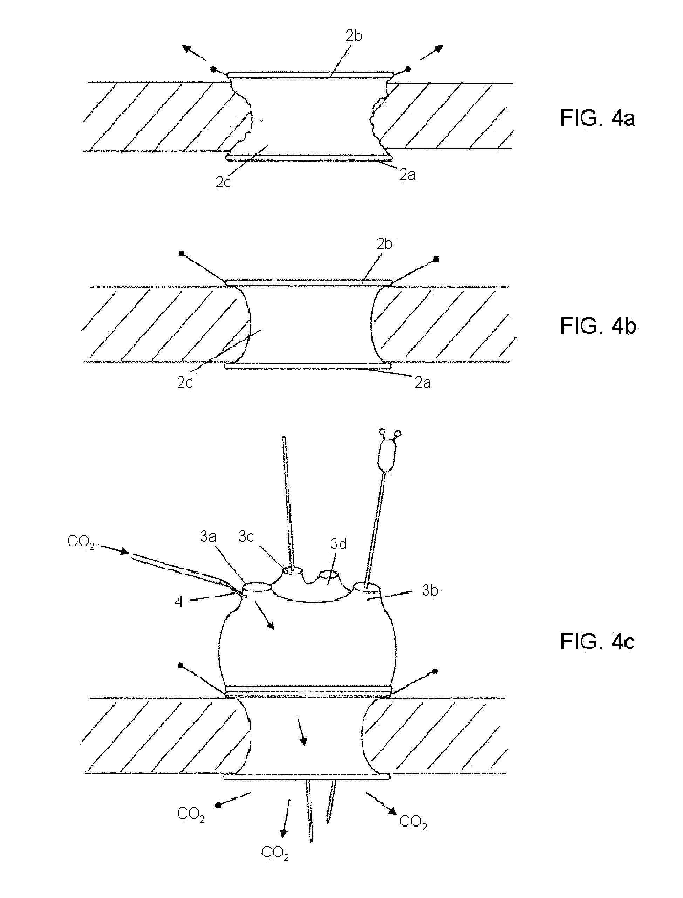

Thus, for using port (1) of the invention, the deformable ring (2a) is firstly deformed until a size enough for inserting it through an insertion, commonly of 1,5-3 cm. Once inside the flexible ring (2a), as shown in figure Fig. 4a, it is necessary to tension the sleeve (2c) pulling the cords (as shown by the arrows of Fig. 4a), being the wall protector (2) finally installed, as in Fig. 4b.

Next, the entry part (3) is connected to the wall protector (2). To this end, in this example, the entry part (3) is provided in its internal portion with a short threaded section which is coupled to a corresponding thread provided inside the stiff ring (2b). In order to avoid a gas escape, this connection may have a rubber O-ring or any similar element.

Finally, once connected to the entry part (3) and to the wall protector (2), CO₂ is injected through the universal female terminal (4) in order to perform the pneumoperitoneum coupled to the valve (3a). The gas entry will then make the "balloon" forming part (3) to swell, becoming completely operative for the insert of the necessary surgical material into any of the valves (3a, 3b, 3c, 3d). It should be noted that, thanks to the high elasticity of the material forming the entry part (3), the maneuverability achieved is much higher than in previous devices.

## Claims

1. Laparoscopy port (1), **characterized in that** it comprises:
- a wall protector (2) formed by a deformable ring (2a) and a stiff ring (2b) joined together by a sleeve (2c) of flexible and puncture-proof material, which is firmly joined to the deformable ring (2a) and slidably joined to the stiff ring (2b); and
- an entry part (3) made of a flexible material capable of swelling by the injection of a gas for the pneumoperitoneum and provided with several valves (3a, 3b, 3c, 3d) for the insertion of surgical material, and which is able to be coupled to the rigid ring (2b) of the wall protector (2).

2. Port (1) according to claim 1, wherein the sleeve (2c) is made from a material based in plastic, polyurethane, polyethylene, nitrile or silicone.

3. Port (1) according to any of the previous claims, wherein the thickness of the sleeve (2c) is between 2 mm and 4 mm.

4. Port (1) according to any of the previous claims, wherein the entry part (3) is made from a material based in plastic, polyurethane, polyethylene, nitrile or silicone.

5. Port (1) according to any of the previous claims, wherein the thickness of the entry part (3) is between 1 mm and 3 mm.

6. Port (1) according to any of the previous claims, wherein the entry part (3) comprises at least one valve (3a) provided with a universal female terminal (4) suitable for connecting a laparoscopy insufflator.

7. Port (1) according to any of the previous claims, comprising four valves.

8. Port (1) according to claim 7, wherein valves (3a, 3b, 3c, 3d) have the following diameters: three valves (3a, 3b, 3c) of 5-12 mm and one valve (3d) of 10-15 mm.

9. Port (1) according to any of the previous claims, wherein the coupling between the wall protector (2) and the entry part (3) is made by means of a threaded or a pressure connection.

10. Port (1) according to any of the previous claims, wherein rings (2a, 2b) of the wall protector (2) have a diameter of between 6 cm. and 10 cm.

11. Port (1) according to claim 10, wherein rings (2a, 2b) have a diameter of 9 cm.

12. Port (1) according to any of the previous claims, which also comprises means for tensioning sleeve (2c).

13. Port (1) according to claim 12, wherein the means for tensioning sleeve (2c) comprise cords joined to the sleeve (2c) and which pass through the stiff ring (2b).
